# EUROPEAN PATENT APPLICATION

(11) **EP 1 441 222 A2**
(43) Date of publication of application: **28.07.2004**
(21) Application number: 03027519.2
(22) Date of filing: 29.11.2003
(51) Int. Cl.: G01N 29/02, G01F 1/66

(54) **Apparatus for and method of acoustically monitoring a gas supply**

(30) Priority: 23.01.2003 SE 0300160
(71) Applicant: Maquet Critical Care AB, 171 95 Solna (SE)
(72) Inventor: Wallen, Lars, 16347 Spänga (SE)

(57) **Abstract**

An apparatus for monitoring a gas supply connection to a mechanical breathing aid (2) comprises in-line housing (14) containing a measurement chamber(20) for receiving gas from the gas supply and a transceiver arrangement (22,24) operable to emit acoustic energy into and to detect emitted acoustic energy propagated through the measurement chamber (20). A comparator (28) is operably connected to the transceiver arrangement(22,24) to receive therefrom a signal dependent on an amplitude of detected energy and is adapted to generate a control signal from a comparison of the received signal with a reference signal indicative of a target gas usable by a flow control valve (32), preferably also located within the housing (14), to inhibit a gas flow through the breathing aid (2).

## Description

The present invention relates to an apparatus for and method of monitoring a gas supply and in particular to an apparatus and method employing ultrasonic energy to monitor for incorrect gas supply connections to a mechanical breathing aid.

Mechanical breathing aids are employed in the administration of a breathing gas to a patient, particularly in a hospital environment, and operate to control either or both the amount and the composition of the administered breathing gas. As such, the term "mechanical breathing aid" encompasses, in the present context, ventilators, respirators and anaesthesia machines as well as on-demand type face masks employed in medical environments. External gas supplies, such as may be provided locally by pressurised bottles or remotely by a central storage facility or both, connect to these breathing aids to act as one or more sources of gas making up the breathing gas. Typically, there is often more gas supplies available for connection to the breathing aid than are required and care must be taken to ensure that the correct supplies are connected.

According to a first aspect of the present invention there is provided an apparatus for monitoring a gas supply connection to a mechanical breathing aid as described in and characterised by the present Claim 1 and that may be conveniently configured as an in-line unit. By monitoring the amplitude of acoustic, typically ultrasonic, energy after its passage through gas from a supply connected to the breathing aid a determination of the damping properties of the connected gas can be made and a comparison with those properties of a target gas undertaken. In this manner the gas source connected to the breathing aid can be monitored and gas flow to and/or from the breathing aid automatically stopped if an incorrect gas supply connection is detected.

According to a second aspect of the present invention there is provided a method of monitoring a gas supply connection to a mechanical breathing aid by which method the apparatus according to the first aspect of the present invention operates.

Exemplary embodiments of an apparatus according to the present invention will now be described with reference to the drawings in the accompanying figures, of which:
Fig.1 shows schematically a first embodiment of an apparatus according to the present invention; and
Fig. 2 shows schematically a patient ventilator according to the present invention.

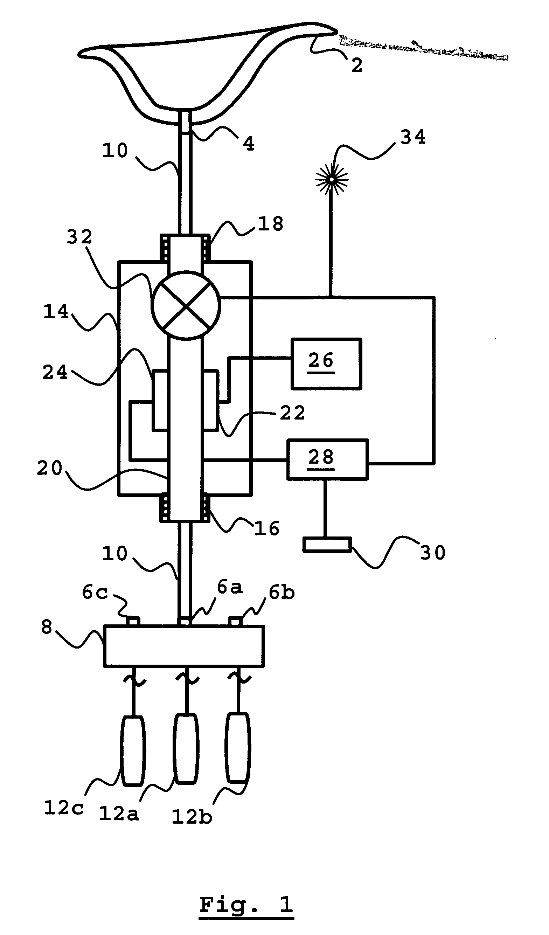

Considering now Fig. 1, a mechanical breathing aid which in the present embodiment is exemplified by an on-demand type face mask 2, has an inlet 4 connected, by means of a gas flow conduit 10, to an outlet 6a of a distribution board 8 for different breathable gases. The distribution board 8 represents a collocation of the outlets of a plurality of different gas supplies 12a,b,c that are typically available in a care environment for selective connection to the inlet 4 of the breathing aid 2 and which, in the present example, are remotely located. In the present example the selective connection is made by manually coupling the gas flow conduit 10 to a selected one of a plurality of outlets 6a,b,c that are connected to the gas supplies 12a,b,c respectively. This selective connection may be realised in other ways such as, for example, by providing a single outlet (6a say) that is switchably connectable to any one of the plurality of gas supplies 12a,b,c.

A housing 14 is, in the present embodiment, removably connectable in-line with the gas flow conduit 10 and is provided with an inlet 16 through which gas from the distribution board 8 is received internal the housing 14 and an outlet 18 through which the received gas flows out of the housing 14. A measurement chamber 20 is located within the housing 14 and connects the inlet 16 to the outlet 18 to permit, in use, a continuous flow of gas through the housing 14, from the supply 12a to the inlet 4 of the face mask breathing aid 2. An ultrasonic transmitter 22 and complementary receiver 24, that in the present example together form a transceiver arrangement, are located within the housing 14 to respectively emit ultrasound energy into the chamber 20 and to detect the emitted ultrasound energy after its propagation though gas within the chamber 20. Whilst in the present example the transmitter 22 and the receiver are shown as being located on opposite sides of the chamber 20 it will be appreciated that the transceiver arrangement 22,24 can be realised in a large number of different ways, for example the transmitter 22 and receiver 24 may be located on the same side of the chamber 20 and an ultrasonic reflector (not shown) located on the opposite side of the chamber 20 to reflect ultrasonic radiation from the transmitter 22 back through the gas within the chamber 20 to the receiver 24, thereby increasing the propagation path of the ultrasound through the gas and thus its absorption by the gas.

A control unit 26 is operably connected to the transmitter 22 to control its emission of ultrasonic energy and a comparator 28 is operably connected to the receiver 24 to receive an output signal from the receiver 24 that is indicative of the amplitude of the ultrasound energy incident upon its detector surface. The comparator may also be, in other embodiments, connected to the control unit 26 to receive a signal indicative of the amplitude of the ultrasound emitted by the transmitter 22. It will be appreciated that one or preferably both the control unit 26 and the comparator 28 may be located within the housing 14. Moreover, a single microprocessor may be configured using known programming techniques and known interface components to perform the functions of both the control unit 26 and of the comparator 28.

The comparator 28 is configured to compare a reference value associated with a target gas which is, in the present example, desired to be present at the inlet 4 of the face mask 2 with a value dependent on the amplitude of the energy received at the receiver 24, such as the amplitude itself or, in other embodiments, a difference value formed from the received signal and the signal representing the amplitude of energy output by the transmitter 22. The comparator 28 is configured to determine from the comparison whether or not there exists a difference between the two amplitudes indicative of the presence of an undesired gas within the conduit 10 and hence of an incorrect gas supply connection to the mechanical breathing aid 2. If the comparator 28 is to be used to monitor for the presence of a predetermined target gas then the reference value may be pre-programmed into the comparator 28. In order to increase its range of use to detect one or more of a plurality of desired gases an input device (30) may be provided by which a user may identify the target gas or gases to the comparator 28 which then operates to adjust the reference amplitude accordingly. This may be achieved by configuring the comparator 28 with a look-up table that indexes reference amplitudes with an alpha-numeric descriptor that uniquely identifies a target gas and that is to be provided to the comparator 28 by the user.

A valve 32 is located, preferably within the housing 14, in the flow path for gas from the distribution board 8 to the face mask 2 and is operable to open or close in order to respectively allow or inhibit gas flow from the connected supply 12a and through the face mask 2 in dependence of a control signal output from the comparator 28. In the event of the comparator 28 making a determination that an incorrect gas supply connection exists then the control signal is output to close the valve 32. In addition or as an alternative to this control signal the comparator 28 may be configured to actuate a sensible warning 34 in the event of such a determination being made.

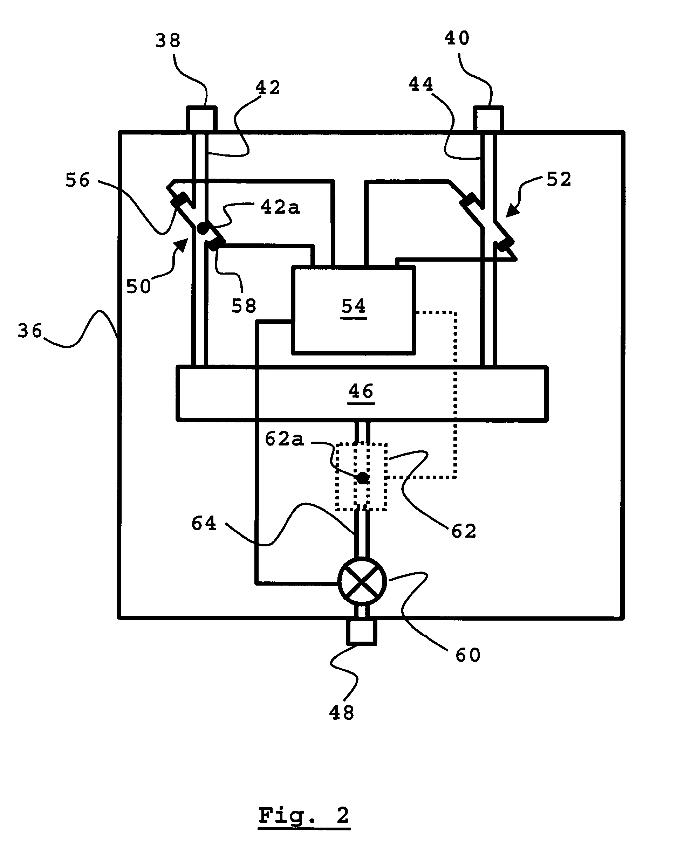

Considering now a patient ventilator 36 that is shown in Fig. 2. In the present example the ventilator 36 is provided with two inlet ports 38,40 intended for connection to a remotely located oxygen supply (not shown) and an air supply (not shown) respectively. Each inlet port 38,40 has, in the present example, an associated gas flow conduit 42,44 internal of the ventilator that connects the associated inlet port 38,40 with a gas mixer 46. The gas mixer 46 is configured in a known manner to provide a correctly proportioned breathing gas mixture at an outlet 48 of the ventilator 36.

Each gas flow conduit 42,44 has provided in flow communication an ultrasonic flow meter 50,52 of a known construction. Each flow meter 50,52 is, in the present example, identical and so the remaining discussion will concentrate only on the flow meter 50 that is located in flow communication with the conduit 42 through which oxygen is intended to flow.

A microprocessor based control unit 54 is provided that is programmed to provide the control and comparison functions described below. The flow meter 50 comprises opposing transceivers 56,58 that are controlled by the unit 54 to operate in a known manner to alternately act as an ultrasound transmitter and a complementary receiver. In a known manner, the unit 54 is configured to measure transit times of ultrasonic energy that travels through gas within the conduit 42, between the transceivers 56,58 and from this determine a flow rate within the conduit 42. The control unit 54 may then vary the flow rates as required to ensure, in a known manner, that a correct proportion of gases enters the gas mixer 46.

In addition to this known configuration the control unit 54 is, according to the present invention, further configured to operate as a comparator. The comparator 54 is arranged to receive an input signal from one or both of the transceivers 56,58 when acting as a receiver that is indicative of the amplitude of the acoustic energy received at the acting receiver. Thus, in the present embodiment, a portion 42a of the existing conduit 42 through which the ultrasound propagates will act as a measurement chamber of the apparatus according to the present invention and the transceivers 56,58 of the flow meter 50 will act as a transceiver arrangement of the above mentioned apparatus.

The comparator 54 is additionally configured to compare the amplitude that is represented by the input signal with a reference amplitude associated with a target gas that, in the present example, is so-called "laughing gas" (N₂O) and that here is not desired to be connected to either of the inlet ports 38,40.

As will be appreciated, laughing gas is a gas supply that is commonly available in most hospital environments where oxygen and air supplies are available and could prove dangerous to a patient if erroneously connected to either one of the inlet ports 38,40 of the ventilator 36. It will be also appreciated that N₂O absorbs ultrasound to a much greater degree than either oxygen or air.

A valve 60 is, in the present embodiment, located in gas communication with the outlet port 48 and can be operated to inhibit breathing gas flow from the ventilator 36 .The comparator 54 is still further configured to generate a control signal to operate the valve 60 to inhibit gas flow in the event that a difference value (for example zero) is determined from the comparison within the comparator 54 of the input amplitude with the reference amplitude that indicates the presence laughing gas within the conduit 42 and hence an incorrect gas supply connection to the breathing aid 36.

The same control signal will also be generated by the comparator 54 dependent of a same comparison made with respect to signals from the flow meter 52 associated with the other conduit 44.

The above described embodiment employs components that typically already exist within a known ventilator 36 and which are suitably adapted to operate according to the present invention. This multiple use of components advantageously permits the incorporation of the apparatus according to the present invention into a mechanical ventilator 36 with a minimum of cost and space increases. However, it will be appreciated that the functions of the flow meters 50,52 and of the control unit 54 which, as described above, relate to their operation as components of the apparatus according to the present invention may be provided by separate, additional components. Some or all of these additional components preferably being collocated within an in-line housing similar to that 14 described with respect to the embodiment of Fig. 1.

In an alternative embodiment of the patient ventilator 36 which is also illustrated in Fig. 2, a transceiver arrangement 62 is located to emit ultrasound into and detect the emitted ultrasound after propagation through gas within a reference chamber portion 64a of a conduit 64 connecting the gas mixer 46 with the outlet port 48 of the ventilator 36. An output signal indicative of the amplitude of the acoustic energy detected by the transceiver arrangement 62 is provided to the control unit 54. In this instance the control unit 54 when acting as a comparator is configured to compare this amplitude with a reference value associated with a gas mixture desired at the outlet port 48 (which value may be determined automatically in a known manner by the control unit 54 in dependence of the flow rates determined by flow meters 50,52 located in the conduits 42,44 associated with the inlet ports 38,40 of the ventilator 36) and to provide a control signal closing the valve 60 to inhibit gas flow from the ventilator 36 in the event that a difference value (for example zero) is determined from the comparison within the unit/comparator 54 of the input amplitude with the reference amplitude indicating an erroneous gas mixture in the conduit 64 and hence an incorrect gas supply connection to one or both of the inlet ports 38,40.

Whilst the apparatus according to the present invention has been described above in connection with the monitoring for connection of N₂O it may be used to monitor the coupling of many other gases into the mechanical breathing aid provided that these other gases can provide a measurable and differentiable attenuation of acoustic energy.

## Claims

1. An apparatus for monitoring a gas supply connection to a mechanical breathing aid (2;36) comprising a measurement chamber(20;42a;64a) for receiving gas from the gas supply and a transceiver arrangement (22,24;56,58;62) operable to emit acoustic energy into and to detect emitted acoustic energy propagated through the measurement chamber (20;42a;64a) **characterised in that** the apparatus further comprises a comparator (28;54) operably connectable to the transceiver arrangement(22,24;56,58;62) to receive a signal dependent on an amplitude of detected energy and is adapted to generate a control signal from a comparison of the received signal usable to inhibit a gas flow through the breathing aid (2;36).

2. An apparatus as claimed in Claim 1 **characterised in that** the comparator (28;54) is adapted generate the control signal dependent on the deviation of a value representing the amplitude of the detected energy from an expected amplitude value associated with a target gas.

3. An apparatus as claimed in Claim 1 **characterised in that** the comparator (28;54) is adapted generate the control signal dependent on the deviation of a value representing the difference between the amplitudes of the emitted and the detected energy from an expected difference value associated with a target gas.

4. An apparatus as claimed in any of the claims 1 to 3 **characterised in that** a valve (32;64) is provided for gas communication with flow conduits (10;42;22;64) connecting the breathing aid (2;36) to the supply (12a,b,c) and adapted to respond to the control signal to inhibit the gas flow.

5. An apparatus as claimed in any preceding claim **characterised in that** the apparatus further comprises a housing (14) having an inlet (16) and an outlet (18) removably connectable in-line with an external gas flow conduit (19) and interconnected by the measurement chamber (20) to define a gas flow path through the housing (14).

6. A mechanical breathing aid (36) having an inlet (38;40) for connection to a supply of gas **characterised in that** the mechanical breathing aid (36) further includes a transceiver arrangement (56,58;62) operable to emit acoustic energy into and detect emitted acoustic energy propagated through a conduit (42;64) in gas connection with the inlet (38;40); and a comparator (54) operably connectable to the transceiver arrangement (56,58;62) to receive a signal therefrom dependent on an amplitude of the detected energy and is adapted to generate a control signal from a comparison of the received signal usable to inhibit a gas flow through the breathing aid (36)

7. A mechanical breathing aid as claimed in Claim 6 **characterised in that** the mechanical breathing aid (36) further includes a valve (60) disposed in gas communication with the conduit (42;64) and operable in response to the control signal to inhibit the gas flow through the mechanical breathing aid (36).

8. A method of monitoring a gas supply connection to an inlet (4;38;40) of a mechanical breathing aid (2;36) comprising the steps of:
emitting acoustic energy into a measurement chamber (20;42a;64a) for receiving gas connected to the inlet (4;38;40) ;
detecting the amplitude of the emitted acoustic energy after propagation through the measurement chamber (20;42a;64a);
generating an indicator of the actual gas within the conduit dependent on the detected amplitude;
comparing within a comparator (28;54) the indicator of the actual gas with an indicator of a target gas; and
generating a control signal usable to inhibit a gas flow through the mechanical breathing aid (2;36) dependent on the comparison indicating an incorrect gas supply connection.
